# EUROPEAN PATENT APPLICATION

(11) **EP 3 766 435 A1**
(43) Date of publication of application: **20.01.2021**
(21) Application number: 19772305.9
(22) Date of filing: 19.03.2019
(51) Int. Cl.: A61B 17/12

(54) **EMBOLUS MATERIAL**

(30) Priority: 22.03.2018 JP 2018054613
(71) Applicant: TERUMO Kabushiki Kaisha, Tokyo 151-0072 (JP)
(72) Inventor: IKUNO, Eri, Ashigarakami-gun, Kanagawa 259-0151 (JP); ITOH, Yuki, Ashigarakami-gun, Kanagawa 259-0151 (JP); SAKAGAWA, Yohichi, Ashigarakami-gun, Kanagawa 259-0151 (JP)
(74) Representative: Casalonga
(86) International application number: PCT/JP2019/011513
(87) International publication number: WO 2019/181959

(57) **Abstract**

There is provided an embolus material (10) for inhibiting the inflow of blood into an aortic aneurysm (92) from a branched blood vessel (94) by filling, via a catheter (100), the inside of the aortic aneurysm (92) joined to the branched blood vessel (94), the embolus material (10) being provided with: an elongated filling body (12) that is formed to be narrower than the inside diameter of the catheter (100); and a shaping section (14) that is provided at a portion of the filling body (12) and that forms a structure larger than the width of the branched blood vessel (94) after exiting from the catheter (100) .

## Description

### Technical Field

The present invention relates to an embolus material used to treat an aneurysm formed in a blood vessel.

### Background Art

In the related art, a method of providing an artificial blood vessel through laparotomy or thoracotomy has been used to treat an aortic aneurysm, but is large invasive treatment and imposes a great burden on a patient. Thus, in recent years, transition to stent graft treatment of performing treatment in a blood vessel has occurred.

The stent graft treatment is treatment that inhibits blood from flowing into the aortic aneurysm by inserting a catheter from the patient's femoral artery and providing an artificial blood vessel (stent graft) with a spring in a target lesion.

However, it is known that the stent graft treatment causes several complications. One of the complications is Type 2 endoleak. Type 2 endoleak is a complication in which a blood flow that was flowing out in a forward direction from a branch vessel located in the aortic aneurysm part to a terminal blood vessel is reversed from the terminal blood vessel side to the inside of the aneurysm due to the provision of a stent graft, and thus the blood flow remains in the aneurysm. If this is left unattended for a long period of time, the aortic aneurysm may be enlarged by the reversed blood flow. Such Type 2 endoleak is said to occur in about 20% to 30% of patients who have undergone stent graft treatment in the long term.

As a method for treating Type 2 endoleak, a method has been proposed in which the origin of a branch vessel that is branched from an aortic aneurysm is occluded with an embolus coil. A method of filling a liquid embolus material into an aortic aneurysm has been proposed in "A Novel and Simple Technique for Embolization of Type 2 Endoleaks Through Direct Sac Access From the Distal Stent-graft Landing Zone", G. Coppi et-al., European Journal of Vascular and Endovascular Surgery, Volume 47 Issue 4 p. 394 to 401, April/2014. JP-T-2002-539853 and JP-T-2004-537353 disclose a method of filling a gelatinous embolus material.

### Summary of Invention

However, there may be a plurality of branch vessels connected to an aortic aneurysm. Since some branch vessels are thin or complicated, it is hard to access all branch vessels, and it is difficult to occlude all branch vessels with an embolus coil. Currently, treatment of occluding a main branch vessel connected to an aortic aneurysm is performed, but there is a problem in that inflow of blood from a branch vessel that cannot be treated is not prevented, and the recurrence rate is high.

In the treatment method using a liquid embolus material, a liquid embolus material that is solidified when contact with blood is injected into an aortic aneurysm, and thus all blood vessels connected to the aortic aneurysm are blocked.

However, when the liquid embolus material is injected into the aortic aneurysm, the liquid embolus material before being solidified flows out to a peripheral portion of a branch vessel and distally embolizes a blood vessel at a part separated from the aortic aneurysm, and thus there is a problem in that a new complication is caused.

Therefore, as a method of blocking all blood vessels connected to an aortic aneurysm without accessing all branch vessels, it is considered that an elongated embolus material that is fillable via a catheter is filled into the aortic aneurysm, but an embolus material that is fillable via a catheter may be thinner than some branch vessels connected to the aortic aneurysm. In this case, the embolus material flows out to a peripheral portion of a branch vessel in the same manner as a liquid embolus material, and thus there is concern that a blood vessel at a part separated from the aortic aneurysm is distally embolized.

An object of the present invention is to provide an embolus material capable of inhibiting blood from flowing into an aortic aneurysm while preventing the occurrence of a complication due to the entire embolus material flowing out of a branch vessel connected to the aortic aneurysm.

In order to achieve the object, there is provided an embolus material according to an aspect of the invention that is configured to be filled into an aortic aneurysm connected to a branch vessel via a catheter to inhibit inflow of blood into the aortic aneurysm from the branch vessel or outflow of the blood from an inside of the aortic aneurysm to the branch vessel, the embolus material including an elongated filling body that is formed to be smaller than an inner diameter of the catheter; and a shaped portion that is provided at least at a part of the filling body and is configured to be bent or curved after coming out of the catheter, in which the shaped portion is configured to spread out, in a bent or curved state, wider than an inner diameter of the branch vessel.

When an aneurysm is accessed from a branch vessel, or when the aneurysm is accessed through transcealing, a thickness of a catheter may be restricted due to difficulty in the access, and thus a small embolus material may have to be injected from a catheter smaller than an inner diameter of a branch vessel. Even in such a case, according to the embolus material described above, since the shaped portion having a width larger than that of the branch vessel is provided in a part of the embolus material, it does not occur that the entire embolus material strays into the branch vessel and thus occludes an unintended location. Consequently, it is possible to prevent a reverse flow of blood into an aneurysm without causing a new complication. In a case where an embolus material smaller than an inner diameter of a branch vessel, an effect that a portion other than the shaped portion enters the branch vessel and thus directly embolizes the branch vessel can be expected.

In the embolus material, the filling body may be made of a hydrogel that swells through contact with blood. Here, the hydrogel is a polymer material that swells through contact with water or a solvent and thus increases in volume. The hydrogel also includes a stimulus-responsive gel material of which a volume increases in response to stimuli such as pH, a temperature, water, and an ionic strength. Such an embolus material does not adhere to a tip of a catheter unlike a liquid embolus material, and thus can be easily handled by using the catheter. The filling body swells after being provided, and thus an amount of the provided embolus material is small. The embolus material does not entirely bury a blood vessel aneurysm immediately after being provided, gradually buries the aneurysm by swelling, and is thus excellent in safety without causing a sudden pressure increase.

In the embolus material, the shaped portion may include a shape memory member wound in a coil shape on an outer circumferential portion of the filling body. The shape memory member is a member deformed into a predetermined shape due to a temperature, a solvent (water), pH, an ionic strength, or the like. Such a shape memory member is shaped into a predetermined shape in advance, and thus a terminal end of a filling body discharged from a catheter can be deformed to form a structure larger than a branch vessel. As a result, it is possible to prevent an embolus material from flowing into a branch vessel.

In the embolus material, the shaped portion may include a shape memory member buried inside the filling body. It is possible to form a structure larger than an inner diameter of a branch vessel at a terminal portion of the filling body by using the shape memory member. Consequently, it is possible to prevent the embolus material from flowing into a branch vessel.

In the embolus material, the shaped portion may be shaped through polymerization in a mold having a shape of the structure. Since a crosslinking structure of molecules forming the filling body reflects a shape in the mold at the time of synthesis, a pre-shaped shape is shown when the provided filling body swells. Consequently, it is possible to prevent an embolus material from flowing into a branch vessel.

In the embolus material, the shaped portion may be formed of an elastic member or a shape memory member coupled to a terminal end of the filling body. Consequently, it is possible to prevent an embolus material from flowing into a branch vessel.

In the embolus material, the shaped portion may be formed at both ends of the filling body. The shaped portion may be formed over an entirety of the filling body. It is also possible to prevent an embolus material from flowing into a branch vessel by using the configurations. Since the terminal end of the embolus material is shaped, the risk of the distal end thereof sticking to an aneurysm portion and puncturing the aneurysm portion can be reduced.

In the embolus material, the shaped portion may be shaped so as to spread out in a range wider than the inner diameter of the branch vessel in a width direction of the filling body by bending with respect to the filling body. Consequently, it is possible to prevent an embolus material from flowing into a branch vessel.

In the embolus material, the shaped portion may be shaped into a circular or polygonal ring shape having a diameter larger than the inner diameter of the branch vessel. The shaped portion may be shaped into an umbrella shape having a diameter larger than the inner diameter of the branch vessel by virtue of terminal ends of the branched filling body being turned back to a proximal side of the filling body. The shaped portion may be shaped such that terminal ends of the branched filling body forms a ball-like shape having a diameter larger than the inner diameter of the branch vessel. Such a shaped portion can also prevent an embolus material from entering a branch vessel.

The embolus material is used to treat an aortic aneurysm, and the shaped portion may be configured to forma structure having a diameter of 3 mm or more. Consequently, since a width of the structure of the shaped portion is larger than an inner diameter of a branch vessel connected to an aortic aneurysm, for example, the inferior mesenteric artery having a thickness of about 3 mm or the lumbar artery having a thickness of about 2 mm, it is possible to prevent the embolus material from entering the branch vessel. Such an embolus material is suitable for treating Type 2 endoleaks.

In the embolus material, the filling body may have a diameter smaller than the inner diameter of the branch vessel in a state before swelling such that a part of the filling body other than the shaped portion may enter and occlude the branch vessel. Consequently, the embolus material is filled into an aneurysm, and an effect that a portion other than the shaped portion enters a branch vessel and thus directly embolizes the branch vessel can also be expected.

In the embolus material, a linear portion not being shaped may be disposed at a terminal portion of the filling body. According to the embolus material, the linear portion can easily enter a branch vessel, and thus an effect that the linear portion having entered the branch vessel occludes the branch vessel can be expected.

The embolus material according to the present invention can inhibit blood from flowing into an aortic aneurysm while preventing the occurrence of a complication due to the entire embolus material flowing out of a branch vessel connected to the aortic aneurysm.

### Brief Description of Drawings

[Fig. 1] Fig. 1 is a perspective view illustrating an embolus material according to a first embodiment of the present invention.
[Fig. 2] Fig. 2 is a schematic configuration diagram illustrating an example of using the embolus material in Fig. 1 for an aortic aneurysm.
[Fig. 3] Fig. 3 is a schematic configuration diagram illustrating an example of an operation of the embolus material in Fig. 1.
[Fig. 4] Fig. 4 is a perspective view illustrating an embolus material according to a second embodiment of the present invention.
[Fig. 5] Fig. 5A is a plan view illustrating an embolus material according to a third embodiment, Fig. 5B is a cross-sectional view taken along the line VB-VB in Fig. 5A, and Fig. 5C is a cross-sectional view taken along the line VC-VC in Fig. 5A.
[Fig. 6] Fig. 6 is a perspective view illustrating an embolus material according to a fourth embodiment.
[Fig. 7] Fig. 7A is a plan view illustrating a part of a mold used to manufacture the embolus material in Fig. 6, Fig. 7B is a plan view illustrating a state in which a raw material for the embolus material flows into the mold in Fig. 7A, and Fig. 7C is a plan view illustrating the embolus material formed by the mold in Fig. 7A.
[Fig. 8] Fig. 8 is a perspective view illustrating a terminal portion of an embolus material according to a fifth embodiment of the present invention.
[Fig. 9] Fig. 9 is a perspective view illustrating a terminal portion of an embolus material according to a sixth embodiment of the present invention.
[Fig. 10] Fig. 10 is a side view illustrating a terminal portion of an embolus material according to a seventh embodiment.
[Fig. 11] Fig. 11A is a perspective view illustrating a first configuration example of a shaped portion in Fig. 10, Fig. 11B is a perspective view illustrating a second configuration example of the shaped portion in Fig. 10, Fig. 11C is a perspective view illustrating a third configuration example of the shaped portion in Fig. 10, and Fig. 11D is a perspective view illustrating a fourth configuration example of the shaped portion in Fig. 10.
[Fig. 12] Fig. 12A is a perspective view illustrating a fifth configuration example of the shaped portion in Fig. 10, and Fig. 12B is a perspective view illustrating a sixth configuration example of the shaped portion in Fig. 10.
[Fig. 13] Fig. 13 is a side view illustrating a seventh configuration example of the shaped portion in Fig. 10.
[Fig. 14] Fig. 14A is a perspective view illustrating a shaped portion of an embolus material according to an eighth embodiment of the present invention, and Fig. 14B is a perspective view illustrating a modification example of the shaped portion in Fig. 14A.
[Fig. 15] Fig. 15 is a perspective view illustrating a shaped portion of an embolus material according to a ninth embodiment of the present invention.
[Fig. 16] Fig. 16 is a perspective view illustrating a shaped portion of an embolus material according to a tenth embodiment of the present invention.
[Fig. 17] Fig. 17 is a perspective view illustrating an embolus material according to an eleventh embodiment of the present invention.
[Fig. 18] Fig. 18 is a perspective view illustrating a shaped portion of an embolus material according to a twelfth embodiment of the present invention.

### Description of Embodiments

Hereinafter, preferable embodiments of the present invention will be described in detail with reference to the drawings.

### (First Embodiment)

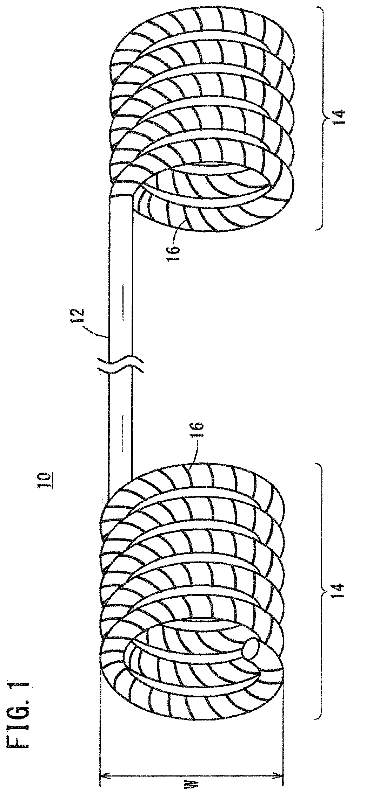

As illustrated in Fig. 1, an embolus material 10 according to the present embodiment includes an elongated filling body 12 forming an embolus material main body, and shaped portions 14 provided at both ends of the filling body 12. The embolus material 10 is provided in an aneurysm such as an aortic aneurysm via a catheter, and the shaped portions 14 form coil-shaped structures. Hereinafter, details of the embolus material 10 will be described.

The filling body 12 is formed in a solid thread-like shape, and the entire length thereof is, for example, about 10 cm to 100 cm. A diameter of the filling body 12 is slightly smaller than an inner diameter of a catheter used to access an aortic aneurysm. The diameter thereof may be, for example, about 1 mm. The filling body 12 is not limited to a solid thread-like shape, and may be formed in a hollow tubular shape. A cross-sectional shape of the filling body 12 is not limited to a circular shape, and may be an elliptical shape, a triangular shape, a rectangular shape, or a polygonal shape, and a combined shape thereof.

The filling body 12 is made of a polymer material that swells by water in blood, and is configured to be swollen after being provided in an aortic aneurysm and thus to be filled into the aortic aneurysm. As such a polymer material, a hydrogel may be used. The hydrogel is manufactured by reacting a mixture containing (a) a monomer or polymer at least a part of which is sensitive to environmental changes such as a pH value, a temperature, water, and an ionic strength, (b) a crosslinking agent, and (c) a polymerization initiator.

As (a) the monomer or polymer, for example, ethylenically unsaturated monomers (for example, acrylic acid-based monomers or amine-based monomers, N,N'-methylenebisacrylamide, N-vinylpyrrolidinone, 2-hydroxyethyl methacrylate, and N-isopropylacrylamide), monomers containing polyols or polyglycols, and derivatives thereof may be used. In addition, a hydrogel composed of a polysaccharide polymer may be used.

The filling body 12 swells through contact with blood by using the composition, and is expanded to a size of 2 to 4 times in a radial direction. A diameter of the filling body 12 after being expanded is about 2 mm to 4 mm. Since this value is equal to or greater than about 2 mm corresponding to an inner diameter of the lumbar artery that is a typical branch vessel connected to an aortic aneurysm, or about 3 mm corresponding to an inner diameter of the inferior mesenteric artery, there is little concern that the filling body 12 after being expanded will stray into the branch vessel. However, since the filling body 12 before being expanded has a diameter of about 1 mm and is thus small, there is concern that the filling body may stray into a large branch vessel.

Therefore, the embolus material 10 of the present embodiment is provided with the shaped portions 14 forming structures each having a diameter larger than an inner diameter of a branch vessel at terminal portions of the filling body 12. The shaped portion 14 forms a structure that has a linear shape in the same manner as other portions of the filling body 12 in a catheter, but is deformed into a predetermined shape after being discharged from the catheter, and thus has a size not to enter a branch vessel, at the terminal portion of the filling body 12.

In the present embodiment, the shaped portion 14 is shaped such that the terminal portion of the filling body 12 forms a structure curved in a coil shape. A diameter W of the coil-shaped portion is more than about 2 mm to 3 mm corresponding to an inner diameter of a branch vessel. Therefore, in the filling body 12 used to treat an aortic aneurysm, the diameter W is preferably 3 mm or more. In a case where a curvature radius of the curve of the structure is too large, the terminal portion of the filling body 12 easily enters a branch vessel in the same manner as the linear filling body 12, and thus the curvature radius of the curve is preferably equal to or less than twenty times the inner diameter of the branch vessel.

The shaped portion 14 of the present embodiment is formed by winding a thin wire 16 formed of a shape memory member in a coil shape on an outer circumference of the filling body 12. A diameter (wire diameter) of the thin wire 16 may be about 0.01 to 0.5 mm, and may employ a wire thinner than the filling body 12.

As the shape memory member forming the thin wire 16, for example, a shape memory alloy or a shape memory resin may be used. As the shape memory alloy, for example, Ni-Ti-based alloys (Nitinol), Cu-Al-Ni-based alloys, Cu-Sn-based alloys, Cu-Zn-based alloys, Cu-Zn-X (X=Si, Al, or Sn)-based alloys, Fe-Pt-based alloys, Mn-Cu-based alloys, Fe-Mn-Si-based alloys, Fe-Ni-Co-Al-based alloys, Pt-based shape memory alloys, Co-Ni-Al-based alloys, Co-Ni-Ga-based alloys, Ni-Fe-Ga-based alloys, and Ti-Pd-based alloys may be used.

Regarding the shaped portion 14, the terminal portion of the filling body 12 on which the thin wire 16 is wound is shaped in advance into the illustrated coil shape, and then the shaped portion 14 is provided in a state in which the shaped portion is linearly stretched. The embolus material 10 coming out of a catheter forms the illustrated structure as a result of the thin wire 16 of the shaped portion 14 being deformed into the pre-shaped coil shape at a body temperature.

The embolus material 10 of the present embodiment has the above-described configuration, and, hereinafter, an operation thereof will be described.

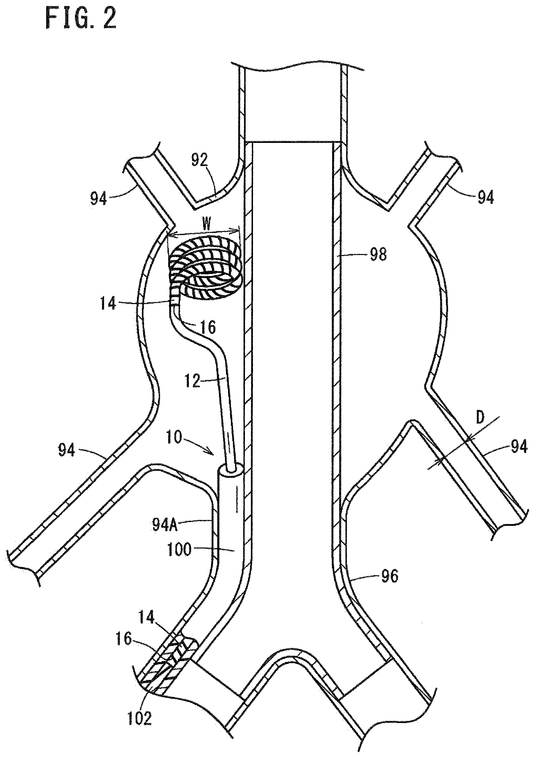

As illustrated in Fig. 2, the vicinities of normal blood vessels 94A located above and below an aortic aneurysm 92 are blocked by a stent graft 98 provided inside a blood vessel 96. The aortic aneurysm 92 is connected to a plurality of branch vessels 94 toward terminal portions such as the leg, the bowel, and the spinal cord. The embolus material 10 can be introduced into the aortic aneurysm 92 via a catheter 100 provided between the stent graft 98 and the blood vessel 96. The catheter 100 may be provided in advance between the stent graft 98 and the blood vessel 96 when the stent graft 98 is provided.

For example, as disclosed in JP-T-2002-539853, after the stent graft 98 is provided, a method may be employed in which the catheter 100 is inserted while making a gap between the stent graft 98 and the blood vessel 96, to secure access to the aortic aneurysm 92. There may be a method of accessing the aortic aneurysm 92 by causing the catheter 100 to pass through the branch vessel 94 connected to the aortic aneurysm 92. There may be a method of directly inserting the catheter 100 into the aortic aneurysm 92 by directly puncturing the aortic aneurysm 92.

The thread-like embolus material 10 of which the terminal portion is shaped in advance into a predetermined shape is inserted into the catheter 100 that is inserted into the aortic aneurysm 92. The embolus material 10 is pushed out by a pushout member 102 inserted from a proximal portion side of the catheter 100, and is provided in the aortic aneurysm 92 from a distal end of the catheter 100. Thereafter, the catheter 100 is extracted from the aortic aneurysm 92 and the blood vessel 96.

In the embolus material 10 provided in the aortic aneurysm 92, the thin wire 16 made of a shape memory alloy wound on the terminal portion of the filling body 12 is deformed into a pre-shaped coil shape due to a body temperature. As a result, a structure with a width W more than an inner diameter D of the branch vessel 94 is formed at the terminal portion of the embolus material 10. Consequently, the embolus material 10 is prevented from flowing into the branch vessel 94. The filling body 12 of the embolus material 10 is gradually swollen by water in blood, to be filled into the inside of the aortic aneurysm 92. The embolus material 10 may be provided a plurality of times until the swollen filling body 12 is filled into the inside of the aortic aneurysm 92.

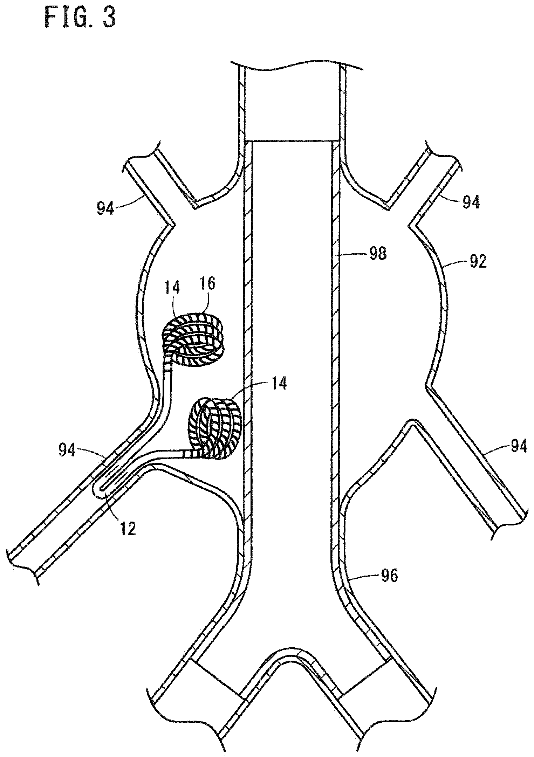

The present inventors have made tests and found that a portion other than the shaped portion 14 enters the branch vessel 94 and thus partially embolizes the branch vessel 94. In other words, as illustrated in Fig. 3, it is clear to achieve an effect that the linear portion of the filling body 12 enters the branch vessel 94 in a bent state, to be then swollen in the branch vessel 94, and thus directly embolizes the branch vessel 94. Even in this case, since the shaped portion 14 is formed in the embolus material 10, the entire embolus material 10 does not distally flow out to the branch vessel 94, and there is little concern of causing a complication. As mentioned above, according to the embolus material 10 of the present embodiment, it can be expected to achieve an effect that the embolus material fills the aortic aneurysm 92 and also directly embolizes the branch vessel 94.

As mentioned above, according to the embolus material 10 of the present embodiment, the embolus material can be shaped to spread out in a range wider than the inner diameter D of the branch vessel 94 in the width direction of the filling body 12. The shaped portion 14 can prevent the entire embolus material 10 from entering the branch vessel 94. As a result, the inside of the aortic aneurysm 92 or the branch vessel 94 can be filled with the embolus material 10 and thus it is possible to inhibit blood from flowing into the aortic aneurysm 92 while preventing the occurrence of a complication caused by embolizing a blood vessel at a part separated from the aortic aneurysm 92.

The filling body 12 forming the embolus material 10 is made of a hydrogel that swells through contact with blood, and thus an amount of the provided embolus material 10 may be small. In this case, since the embolus material is not filled into the aortic aneurysm 92 or the branch vessel 94 immediately after being provided but swells to be gradually buried into the aortic aneurysm 92 or the branch vessel 94, the embolus material does not cause a sudden pressure increase in the aortic aneurysm 92 or the branch vessel 94 and can thus be provided safely.

The filling body 12 of the embolus material 10 is made of a flexible thread-like gel material, and can thus follow the aortic aneurysm 92 or the branch vessel 94 even in a case where a shape thereof is deformed. The filling body 12 of the embolus material 10 before swelling does not adhere to the tip of the catheter 100 in the same manner as a liquid embolus material, and thus the catheter 100 can be easily handled and be easily provided.

Since the terminal end of the embolus material 10 is shaped, the risk of the distal end thereof sticking to the aortic aneurysm 92 and puncturing the aortic aneurysm can be reduced.

### (Second Embodiment)

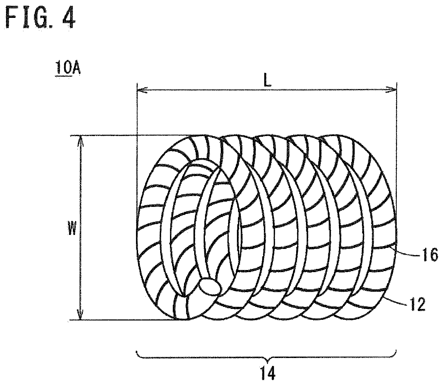

In an embolus material 10A according to the present embodiment, as illustrated in Fig. 4A, the entire filling body 12 is formed in a coil shape. In other words, the shaped portion 14 is provided over an entirety of the filling body 12.

The filling body 12 is made of a thread-like hydrogel in the same manner as the filling body 12 described with reference to Fig. 1, and the thin wire 16 formed of a shape memory member is wound on an outer circumferential portion thereof. In the present embodiment, the thin wire 16 is wound from one end of the filling body 12 to the other end thereof. The thin wire 16 is shaped in advance such that the filling body 12 is deformed into a coil shape as illustrated when coming into contact with a body temperature. The filling body 12 on which the thin wire 16 is wound is provided in a linearly stretched form.

The width W and the entire length L of a coil-shaped structure formed by the shaped portion 14 are preferably more than the inner diameter D of the branch vessel 94. In a case where the embolus material 10A is used in the aortic aneurysm 92, the inner diameter D of the typical branch vessel 94 connected to the aortic aneurysm 92 is about 2 to 3 mm, and thus the width W and the entire length L of the shaped portion 14 before swelling may be equal to or more than, for example, 3 mm.

The embolus material 10A of the present embodiment is inserted into the catheter 100 (refer to Fig. 2) in a state in which the filling body 12 is linearly stretched, and is provided in the aortic aneurysm 92 via the catheter 100. The embolus material 10A after being provided is deformed into the coil shape having the diameter W and the entire length L more than the inner diameter D of the branch vessel 94 as illustrated in Fig. 4. Consequently, it is possible to prevent the entire embolus material 10A from straying into the branch vessel 94. Since the embolus material 10A is shaped into the coil shape, the risk of the distal end thereof sticking to the aortic aneurysm 92 and puncturing the aortic aneurysm can be reduced.

### (Third Embodiment)

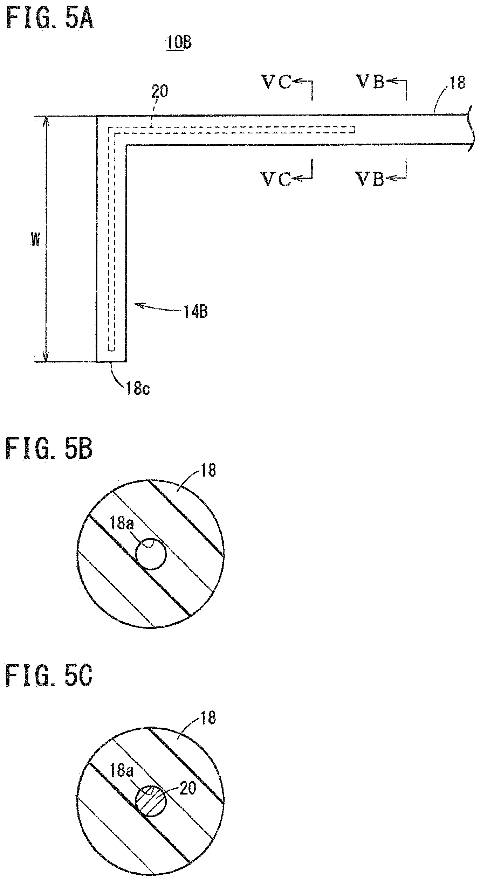

An embolus material 10B according to the present embodiment has a filling body 18 and a shaped portion 14B bent in an L shape at a terminal portion thereof as illustrated in Fig. 5A. As illustrated in Fig. 5B, the filling body 18 is provided with a hollow portion 18a therein. As a material forming the filling body 18, the same material as the material forming the filling body 12 of the embolus material 10 in Fig. 1 may be used.

A core material 20 formed of a shape memory member is inserted into the hollow portion 18a in the vicinity of a terminal end 18c of the filling body 18 as illustrated in Fig. 5C. The core material 20 has a diameter equivalent to an inner diameter of the hollow portion 18a. The core material 20 is made of a shape memory alloy, and is shaped in advance to be bent into an L shape as illustrated when coming into contact with a body temperature.

A shape into which the core material 20 is shaped is not limited to an L shape, and may be a coil shape as illustrated in Fig. 1. The core material 20 may be inserted not only into the vicinity of the terminal portion of the filling body 18 but also into the entire filling body 18 in a total length direction.

The embolus material 10B may be manufactured by shaping the pre-shaped core material 20 into a linear shape at a temperature lower than a body temperature and inserting the core material 20 into the hollow portion 18a from the terminal end 18c of the filling body 18.

When the embolus material 10B is provided in the aortic aneurysm 92 via the catheter 100 (refer to Fig. 2), the core material 20 is deformed into an L shape due to a body temperature. Since the shaped portion 14B deformed into the L shape spreads out in a range W wider than the inner diameter D of the branch vessel 94 connected to the aortic aneurysm 92, the shaped portion 14B is caught at the entrance of the branch vessel 94, and thus it is possible to prevent the entire embolus material 10B from straying into the branch vessel 94. As mentioned above, the embolus material 10B can be filled into the aortic aneurysm 92 or the branch vessel 94 without the entire embolus material straying into the branch vessel 94. Since the terminal end 18c is shaped, the risk of the distal end of the embolus material 10B sticking to the aortic aneurysm 92 and puncturing the aortic aneurysm can be reduced.

In the embolus material 10B of the present embodiment, the filling body 18 is not limited to having the hollow portion 18a, and may be formed in a solid thread-like shape. In this case, the embolus material may be manufactured by placing the linearly stretched core material 20 into a mold after the embolus material is shaped in advance into a predetermined shape and pouring a raw material solution forming a hydrogel around the core material to cause a polymerization reaction to occur such that the core material 20 is buried with the raw material solution.

### (Fourth Embodiment)

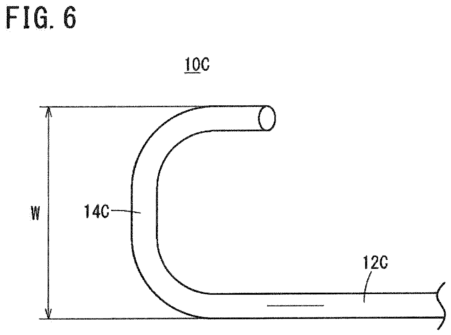

An embolus material 10C according to the present embodiment has a shaped portion 14C formed by shaping a filling body 12C into a predetermined shape as illustrated in Fig. 6. The shaped portion 14C is formed to spread out in the range W wider than the inner diameter D of the branch vessel 94. An example in which the illustrated shaped portion 14C is shaped into a C shape is illustrated, but there is no particular limitation thereto, and the shaped portion may be shaped into an L shape, a corrugated shape, or a coil shape.

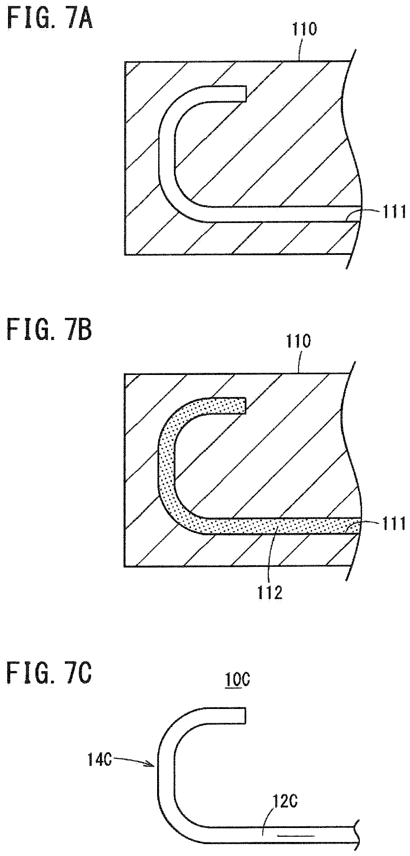

The embolus material 10C of the present embodiment is manufactured according to steps illustrated in Figs. 7A to 7C. First, as illustrated in Fig. 7A, a mold 110 including a cavity 111 having a shape of the shaped portion 14C at a terminal end thereof is prepared.

Next, as illustrated in Fig. 7B, a raw material 112 for a hydrogel forming the filling body 12C is poured into the cavity 111 of the mold 110. In other words, a mixture containing (a) a hydrophilic monomer or polymer, (b) a crosslinking agent, and (c) a polymerization initiator is poured, and a synthetic reaction is performed in the cavity 111 to form the filling body 12C.

Thereafter, as illustrated in Fig. 7C, the filling body 12C synthesized in the cavity 111 is removed from the mold 110, and thus the embolus material 10C of the present embodiment is obtained. In this embolus material 10C, the shaped portion 14C has flexibility and can be stretched to be made linear by applying force thereto. However, since a crosslinking structure of molecules forming the filling body 12C reflects the shape of the shaped portion 14C at the time of synthesis, the filling body naturally returns to the shape in Fig. 7C when swollen.

The embolus material 10C is provided in a state of being dried and linearly stretched. In a case where the embolus material 10C is provided in the aortic aneurysm 92 via the catheter 100, the embolus material naturally returns to an original shape of the shaped portion 14C when swollen. The embolus material 10C can be provided in the aortic aneurysm 92 while preventing the embolus material from entering the branch vessel 94.

As mentioned above, the embolus material 10C of the present embodiment can achieve the same effect as that achieved by the embolus material 10 of the first embodiment.

### (Fifth Embodiment)

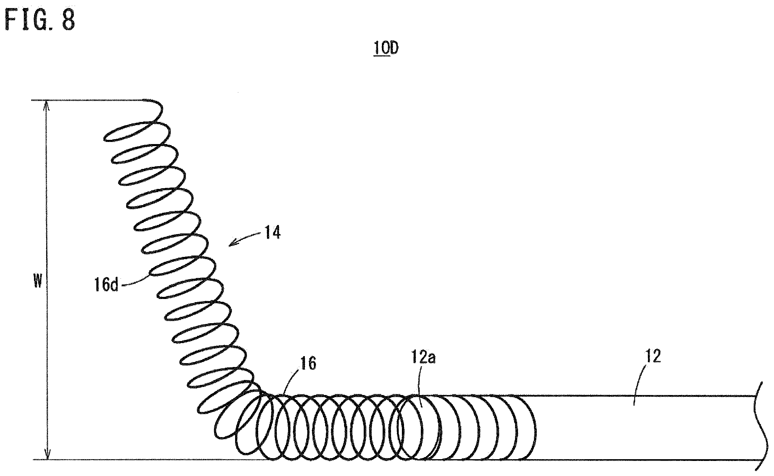

As illustrated in Fig. 8, in an embolus material 10D according to the present embodiment, the thin wire 16 that is shaped into a predetermined shape and is formed of a shape memory member is coupled to the vicinity of an end portion 12a of the filling body 12.

The thin wire 16 is formed in a coil shape, and is coupled to the filling body 12 as a result of a proximal portion thereof being wound on an outer circumferential portion of the elongated filling body 12 around the end portion 12a thereof. An extending portion 16d of the thin wire 16 on the distal side thereof extends from the end portion 12a of the filling body 12 and is shaped in advance to be bent into an L shape as illustrated when coming into contact with a body temperature. The portion bent into the L shape forms the shaped portion 14. Also in the present embodiment, the shaped portion 14 is formed to spread out in the range W wider than the inner diameter D of the branch vessel 94.

The coil-shaped thin wire 16 of the embolus material 10D is shaped into a linear shape along an axis of the filling body 12 at a temperature lower than a body temperature and is provided. Consequently, the embolus material 10D can be smoothly inserted into the catheter 100. In a case where the embolus material 10D is provided in the aortic aneurysm 92 via the catheter 100 (refer to Fig. 2), the embolus material 10D forms a structure in which the extending portion 16d of the thin wire 16 is bent in an L shape due to a body temperature, and thus it is possible to prevent the embolus material 10D from entering the branch vessel 94.

As mentioned above, the embolus material 10D of the present embodiment can achieve the same effect as that achieved by the embolus material 10 of the first embodiment.

### (Sixth Embodiment)

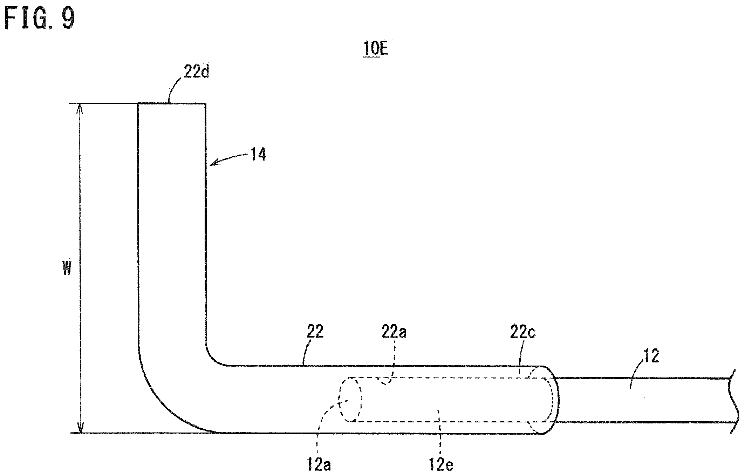

As illustrated in Fig. 9, an embolus material 10E according to the present embodiment includes the filling body 12 and a rod-shaped member 22 attached to the vicinity of the end portion 12a thereof. The rod-shaped member 22 is made of a shape memory alloy, and is shaped in advance such that a distal end 22d side is bent in an L shape to extend. A proximal portion 22c side is formed in a cylindrical shape, and an inner diameter of a concave portion 22a therein is the substantially same as the diameter of the filling body 12.

The distal side 12e of the filling body 12 is inserted into the concave portion 22a, and thus the filling body 12 is coupled to the rod-shaped member 22. The rod-shaped member 22 is provided in a state of being linearly stretched, but is deformed into an L shape as illustrated to form the shaped portion 14 when being provided in the aortic aneurysm 92 and coming into contact with a body temperature. A shape into which the rod-shaped member 22 is shaped is not limited to the L shape, but may be various shapes such as a ring shape, a coil shape, and a spiral shape. The shaped portion 14 is formed to spread out in the range W wider than the inner diameter D of the branch vessel 94 connected to the aortic aneurysm 92.

As mentioned above, the embolus material 10E of the present embodiment can achieve the same effect as that achieved by the embolus material 10 of the first embodiment.

### (Seventh Embodiment)

In the present embodiment, various modification examples of the shaped portions 14, 14B, and 14C of the respective embolus materials 10 to 10E described with reference to Figs. 1 to 9 will be described.

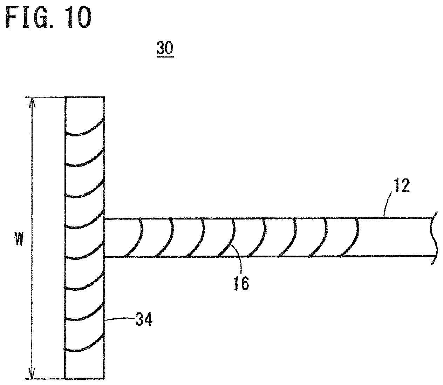

In an embolus material 30 according to a first modification example, as illustrated in Fig. 10, a shaped portion 34 is formed in a plane substantially perpendicular to an axial direction of the filling body 12. In other words, the shaped portion 34 is a two-dimensional structure, and has a T shape as illustrated when viewed from the side. The shaped portion 34 is shaped by the thin wire 16 that is wound in a coil shape on the outer circumferential portion of the filling body 12 and is formed of a shape memory member. The shaped portion 34 may be formed of the filling body 18 (refer to Fig. 5A) into which the core material 20 is built, the filling body 12C (refer to Fig. 7C) shaped by the mold 110, or the rod-shaped member 22 (refer to Fig. 9) instead of the thin wire 16.

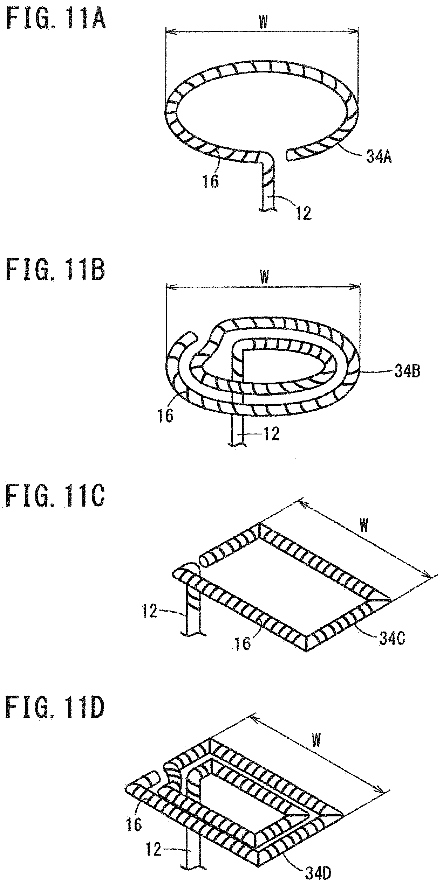

The shaped portion 34 of the embolus material 30 may have shapes as illustrated in Figs. 11A to 12B. A shaped portion 34A according to a first configuration example of the present embodiment illustrated in Fig. 11A is shaped into a circular shape or an elliptical ring shape. A shaped portion 34B according to a second configuration example illustrated in Fig. 11B is shaped into a circular spiral shape or an elliptical spiral shape. The width W of the shaped portions 34A and 34B is larger than the inner diameter D of the branch vessel 94 connected to the aortic aneurysm 92.

A shaped portion 34C according to a third configuration example illustrated in Fig. 11C is shaped into a rectangular ring shape. A shaped portion 34D according to a fourth configuration example illustrated in Fig. 11D is shaped into a rectangular spiral shape. The width W of the shaped portions 34C and 34D in the long side direction is larger than the inner diameter D of the branch vessel 94 connected to the aortic aneurysm 92.

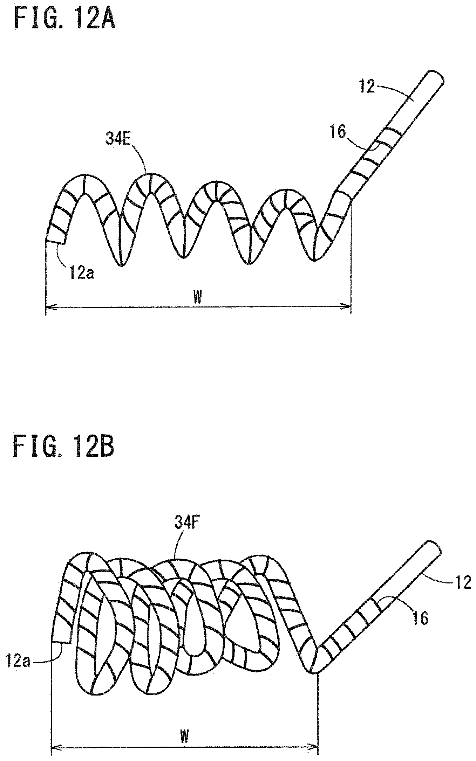

A shaped portion 34E according to a fifth configuration example illustrated in Fig. 12A is shaped into a corrugated shape. A shaped portion 34F according to a sixth configuration example illustrated in Fig. 12B is shaped to be entangled in the same plane. The width W of the shaped portion 34E and 34F is larger than the inner diameter D of the branch vessel 94.

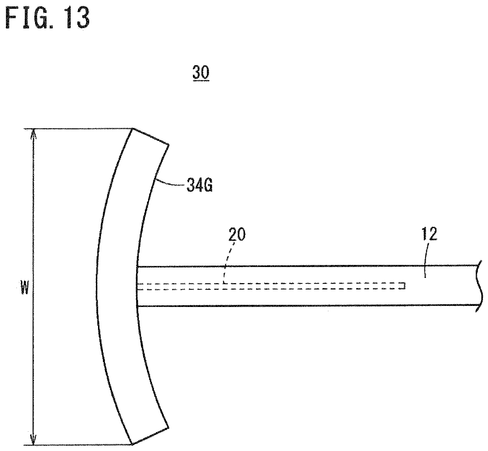

A shaped portion 34G according to a seventh configuration example illustrated in Fig. 13 is shaped to be curved like a bow when viewed from the side. The shaped portion 34G is shaped by the core material 20. A planar shape of the shaped portion 34G may be the same as any one of the shaped portions 34A to 34F illustrated in Figs. 11A to 12B.

The same effect as the effect achieved by the embolus material 10 of the first embodiment can also be achieved by using the first to seventh configuration examples of the present embodiment.

### (Eighth Embodiment)

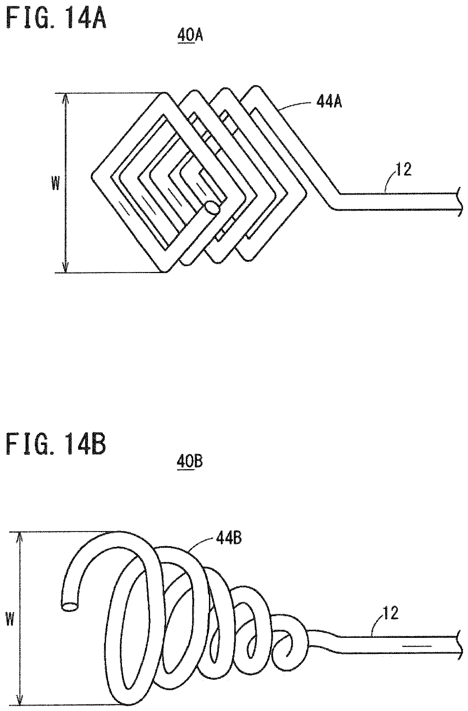

As illustrated in Fig. 14A, an embolus material 40A according to the present embodiment has a shaped portion 44A that is shaped into a rectangular coil shape at a terminal portion of the filling body 12. The shaped portion 44A is formed by a shape memory alloy inserted into the filling body 12. The width W of the shaped portion 44A of the present embodiment is larger than the inner diameter D of the branch vessel 94, and thus it is possible to prevent the embolus material 40A from entering the branch vessel 94.

As illustrated in Fig. 14B, an embolus material 40B according to a modification example of the present embodiment has a shaped portion 44B that is wound in a tornado shape at a terminal portion of the filling body 12. The shaped portion 44B is formed by a shape memory alloy filled into the filling body 12. The width W of the shaped portion 44B of the present modification example is larger than the inner diameter D of the branch vessel 94 connected to the aortic aneurysm 92, and thus it is possible to prevent the embolus material 40B from flowing out to the branch vessel 94. Since the terminal end of the embolus material 40B is shaped, the risk of the distal end thereof sticking to the aortic aneurysm 92 and puncturing the aortic aneurysm can be reduced.

### (Ninth Embodiment)

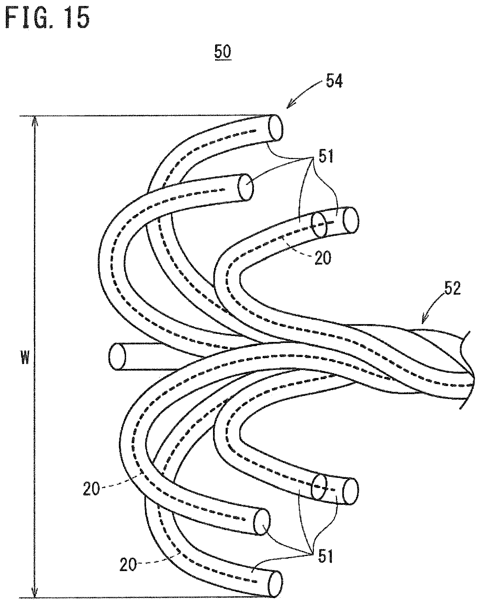

In an embolus material 50 according to the present embodiment, as illustrated in Fig. 15, a filling body 52 is formed by twisting a plurality of filaments 51 made of a hydrogel. A diameter (thickness) of the filling body 52 may be, for example, about 1 mm. The core material 20 shaped in advance into a U shape is inserted into the vicinity of a distal end of each filament 51 forming the filling body 52.

In a case where the embolus material 50 is provided in the aortic aneurysm 92 via the catheter 100, the core material 20 is deformed into the pre-shaped shape due to a body temperature. Consequently, a distal portion of the embolus material 50 is unraveled and the respective filaments 51 are branched, and the distal ends of the filaments 51 spread out to warp toward the proximal sides thereof. As a result, as illustrated, a shaped portion 54 of the present embodiment forms a structure in which the plurality of filaments 51 are developed in an umbrella shape. The shaped portion 54 is shaped such that the width W thereof is wider than the inner diameter D of the branch vessel 94.

For the shaped portion 54, instead of the filament 51 into which the core material 20 is built, the filament 51 on which the thin wire 16 is wound may be used, and a filament shaped in advance by the mold 110 may be used.

As mentioned above, the embolus material 50 of the present embodiment can also achieve the same effect as that achieved by the embolus material 10 of the first embodiment.

### (Tenth Embodiment)

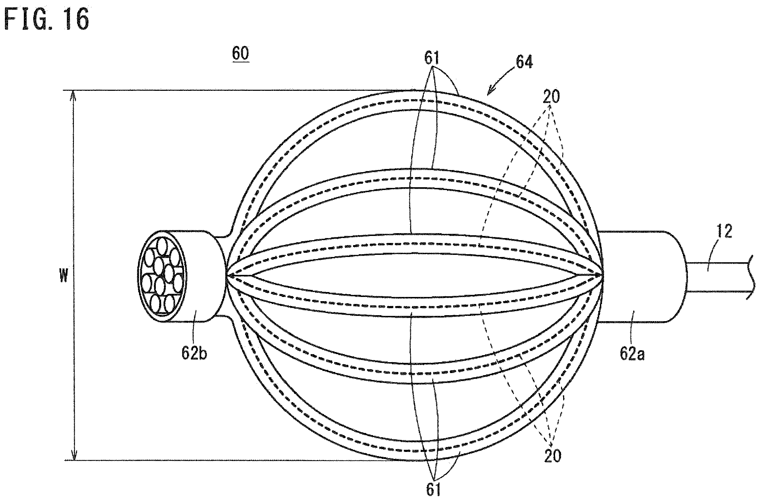

As illustrated in Fig. 16, an embolus material 60 according to the present embodiment includes bundled portions 62a and 62b in which a plurality of filaments 61 made of a hydrogel are bundled as a shaped portion 64. The filaments 61 are bundled at the bundled portion 62a on the proximal side and the bundled portion 62b on the distal side, and spread outwards in a radial direction therebetween.

The core material 20 that is shaped in advance into a C shape to spread outwards in the radial direction is inserted into each filament 61. The core material 20 is made of a shape memory alloy, and is deformed into the pre-shaped shape when coming into contact with a body temperature. The filaments 61 including the core materials 20 are shaped into a linearly bundled shape, and the bundled portion 62a on the proximal side is coupled to the filling body 12.

In a case where the embolus material 60 is provided in the aortic aneurysm 92 via the catheter 100, the respective filaments 61 forming the shaped portion 64 are deformed into a C shape due to a body temperature to form an illustrated ball-like structure. The width W of the shaped portion 64 of the present embodiment is larger than the inner diameter D of the branch vessel 94 connected to the aortic aneurysm 92. For the shaped portion 64, instead of the filament 61 into which the core material 20 is built, the filament 61 on which the thin wire 16 is wound may be used, and a filament shaped in advance by the mold 110 may be used.

As mentioned above, the embolus material 60 of the present embodiment can also achieve the same effect as that achieved by the embolus material 10 of the first embodiment.

### (Eleventh Embodiment)

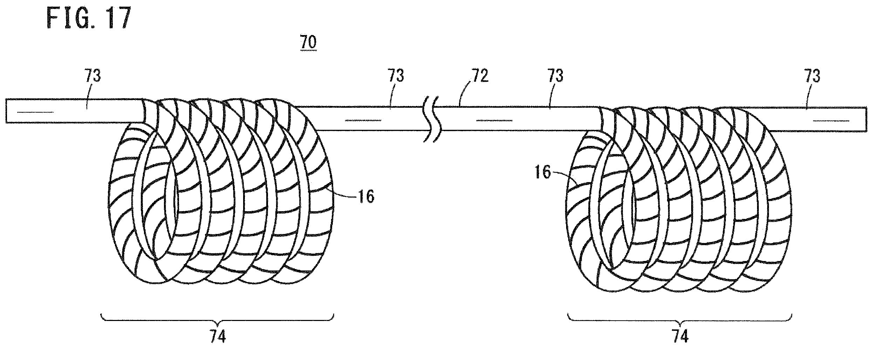

In an embolus material 70 according to the present embodiment, as illustrated in Fig. 17, a shaped portion 74 is provided in a part of a filling body 72, and a terminal end portion thereof is a linear portion 73 that is not shaped. An example is illustrated in which two shaped portions 74 are provided in the illustrated filling body 72, but, the present embodiment is not limited thereto, and the number of shaped portions 74 may be one. The number of shaped portions 74 of the filling body 72 may be three or more. An embolus material may be used in which the shaped portion 74 is provided at one terminal end, and the linear portion 73 is provided at the other terminal portion.

A diameter of the filling body 72 is preferably smaller than the inner diameter D of the branch vessel 94. Consequently, the linear portion 73 easily enters the branch vessel 94. A shape of the shaped portion 74 in the embolus material 70 is not limited to a coil shape, and may have various shapes as illustrated in Figs. 5A to 16. In the filling body 72 having the above-described configuration, the linear portion 73 at the terminal end thereof easily enters the branch vessel 94. The linear portion 73 of the filling body 72 swells to a diameter of 2 to 4 times in the branch vessel 94, and thus an effect that the linear portion 73 directly embolizes the branch vessel 94 can be expected.

### (Twelfth Embodiment)

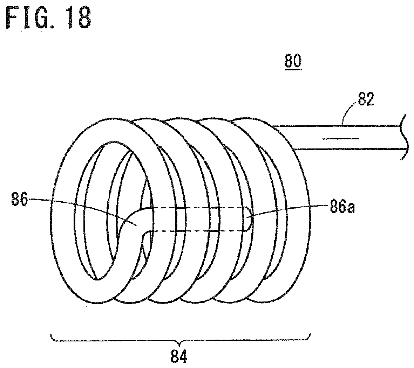

As illustrated in Fig. 18, an embolus material 80 according to the present embodiment has a spiral shaped portion 84 at a terminal portion of a filling body 82. A terminal portion 86 of the shaped portion 84 is shaped to be bent inward of the shaped portion 84, and a tip part 86a of the terminal portion 86 is disposed inside the shaped portion 84.

For the embolus material 80, for example, a raw material solution in which an ethylenically unsaturated monomer, which is a raw material for a hydrogel, a crosslinking agent, and a polymerization initiator are dissolved in a solvent is put in a tube. As this tube, for example, a tube having a predetermined diameter produced from HYTREL (registered trademark) manufactured by DuPont may be used. Such a tube can be dissolved in a predetermined solvent, and the embolus material 80 can be easily taken out from the tube.

Next, the tube of a portion corresponding to the shaped portion 84 of the tube filled with the raw material solution is spirally wound around a hollow pipe-shaped mold. In this case, a terminal portion of the tube is placed inside a spirally wound portion of the mold that is folded back into the hollow portion.

Thereafter, the tube wound around the mold is heated to polymerize the inside solution, and thus a hydrogel is formed to form the embolus material 80. After the hydrogel is formed, the tube is removed with a solvent, the embolus material 80 is washed and is then stretched linearly and dried, and thus the embolus material 80 in a dried and contracted state is obtained.

The embolus material 80 and the manufacturing method thereof according to the present embodiment are as described above, and an operation thereof will be described below.

An embolus material formed by polymerizing a hydrogel in a mold or a tube tends to be softer than an embolus material shaped by a shape memory alloy or the like. When an end part of a shaped portion strays into a branch vessel, such an embolus material may be sucked into the branch vessel from a terminal side all at once, and thus the entire embolus material may stray into the branch vessel.

In contrast, according to the embolus material 80 of the present embodiment, the terminal portion 86 of the embolus material 80 is disposed inside the spiral shaped portion 84. Thus, since the terminal portion 86 does not stray into the branch vessel 94, the embolus material 80 can prevent the shaped portion 84 from being sucked into the branch vessel 94 from the terminal end. Since the terminal end of the embolus material 80 is shaped, the risk of the distal end thereof sticking to the aortic aneurysm 92 and puncturing the aortic aneurysm can be reduced.

In the above description, the present invention has been described by using the preferable embodiments, but the present invention is not limited to the embodiments, and various alterations may occur within the scope without departing from the spirit of the present invention.

## Claims

1. An embolus material configured to be filled into an aortic aneurysm (92) connected to a branch vessel (94) via a catheter (100) to inhibit inflow of blood into the aortic aneurysm (92) from the branch vessel (94) or outflow of the blood from an inside of the aortic aneurysm (92) to the branch vessel (94), the embolus material comprising:
an elongated filling body (12) that is formed to be smaller than an inner diameter of the catheter (100); and
a shaped portion (14) that is provided at least at a part of the filling body (12) and is configured to be bent or curved after coming out of the catheter (100),
wherein the shaped portion (14) is configured to spread out, in a bent or curved state, wider than an inner diameter of the branch vessel.

2. The embolus material according to claim 1,
wherein the filling body (12) is made of a hydrogel that swells through contact with blood.

3. The embolus material according to claim 1 or 2,
wherein the shaped portion (14) includes a shape memory member wound in a coil shape on an outer circumferential portion of the filling body (12).

4. The embolus material according to claim 1 or 2,
wherein the shaped portion (14B) includes a shape memory member buried inside the filling body (12).

5. The embolus material according to any one of claims 1 to 4,
wherein the shaped portion (14) is formed at both ends of the filling body (12).

6. The embolus material according to any one of claims 1 to 4,
wherein the shaped portion (14) is formed over an entirety of the filling body (12).

7. The embolus material according to claim 5,
wherein the shaped portion (14) is shaped so as to spread out in a range wider than the inner diameter of the branch vessel (94) in a width direction of the filling body (12) by bending with respect to the filling body (12) .

8. The embolus material according to claim 5,
wherein the shaped portion is shaped into a circular or polygonal ring shape having a diameter larger than the inner diameter of the branch vessel (94).

9. The embolus material according to claim 5,
wherein the shaped portion (54) is shaped into an umbrella shape having a diameter larger than the inner diameter of the branch vessel (94) by virtue of terminal ends of the branched filling body (52) being turned back to a proximal side of the filling body (52).

10. The embolus material according to claim 5,
wherein the shaped portion (64) is shaped such that terminal ends of the branched filling body (61) forms a ball-like shape having a diameter larger than the inner diameter of the branch vessel (94).

11. The embolus material according to claim 1,
wherein the shaped portion (14) is configured to form a structure having a diameter of 3 mm or more.

12. The embolus material according to claim 1,
wherein the filling body (12) has a diameter smaller than the inner diameter of the branch vessel (94) in a state before swelling such that a part of the filling body (12) other than the shaped portion is able to enter and occlude the branch vessel (94).

13. The embolus material according to claim 12,
wherein a linear portion not being shaped is disposed at a terminal portion of the filling body (12).
